# EUROPEAN PATENT SPECIFICATION

(11) **EP 2 685 946 B1**
(45) Date of publication and mention of the grant of the patent: **03.05.2017**
(21) Application number: 12712021.0
(22) Date of filing: 14.03.2012
(51) Int. Cl.: A61F 5/01

(54) **ORTHOSIS**
ORTHESE
ORTHÈSE

(30) Priority: 14.03.2011 US 201161452557 P
(43) Date of publication of application: 22.01.2014
(73) Proprietor: Cadence Biomedical, Inc., Seattle, WA 98103 (US)
(72) Inventor: SCHOEN, Jason A., Seattle, WA 98112 (US); PACANOWSKY, Alex D., Salt Lake City, UT 84101 (US); GLAISTER, Brian C., Seattle, WA 98103 (US); KAWAHARA, Chie, Seattle, WA 98103 (US); VAN DEN BOGERT, Antonie J., Cleveland Heights, OH 44106 (US); WEST, Zachary, Seattle, WA 98122 (US)
(74) Representative: Teall, Charlotte
(86) International application number: PCT/US2012/029126
(87) International publication number: WO 2012/125765

(56) References cited:
- US-A- 2 267 848
- US-A- 2 536 454
- US-A- 2 573 866
- US-B2- 7 549 969

## Description

### TECHNICAL FIELD

This disclosure generally relates to orthoses and, in particular, to orthoses with one or more exotendons. Document US 7,549,969 B2 is regarded as the closest prior art. This document discloses a system comprising a belt assembly configured to be secured to a user's body, an energy storage apparatus coupled to the belt assembly and including an exotendon, and an articulable leg frame including an upper end rotatably coupled to the belt assembly and a lower end coupleable to a lower portion of a leg of a user. This document is silent as to the presence of an adjustment device which is configured to selectively adjust pretension in the exotendon.

### BACKGROUND

Individuals with injuries or handicaps often wear orthoses that are not energy efficient. Conventional orthoses worn along legs often provide highly unnatural gaits, such as stiff-legged gaits. A user may become rapidly fatigued due to energy inefficiencies and unnatural gaits. Conventional orthoses are also uncomfortable, especially if worn for an extended period of time, because of the unnatural gait and improper positioning of body parts. If an orthosis does not properly position the wearer's foot, the user's leg muscles may become fatigued.

### SUMMARY

The invention is defined in the claims. Some embodiments are directed to an orthosis that can be worn next to a user's leg, prosthesis, or the like. The orthosis or prosthesis can include a framework, rotatable members (e.g., pulleys, guide wheels, etc.), and exotendons extending about the rotatable members. The exotendons can assist the movement of body parts (e.g., leg, foot, etc.) as the user walks, runs, or the like.

In embodiments, an orthosis includes one or more exotendons. The exotendons can include one or more springs, cables, connectors, or the like. The springs
can include stretchable members, helical springs, biasing members, or combinations thereof. In certain embodiments, the exotendon is a multi-piece cable connected together by a tension spring. The tension spring can allow the movement of the exotendon segments to help store energy during exercise (e.g., during the user's gait).

In some embodiments, an orthosis includes a belt assembly, an energy storage apparatus, and an articulatable leg frame. The belt assembly can be secured to a user's body. The energy storage apparatus is couple to the belt assembly and includes an adjustment device and an exotendon. The adjustment device can be configured to selectively adjust pretension, if any, in the exotendon. The leg frame includes an upper end rotatably coupled to the belt assembly and a lower end coupleable to a lower portion of the user's leg. The exotendon is coupled to the adjustment device and to the lower end of the leg frame such that the exotendon stores energy as a leg moves posteriorly and the exotendon releases stored energy to help the leg move anteriorly.

In certain embodiments, the exotendon comprises one or more cables, springs, tethers, or the like. The exotendon can extend along at least a portion of the user's leg. The exotendon can stretch or elongate to store energy and can contract to assist in body movement of the user.

The adjustment device, in some embodiments, includes a one-way ratchet mechanism. The one-way ratchet mechanism can include a gear with teeth that engage a pawl and can be operated by a user to adjust (e.g., increase or decrease) the tension applied to the exotendon. In some embodiments, the adjustment device is capable of changing the tension in the exotendon by at least about 5%, 10%, 20%, 50%, or the like without significantly changing the motion of the user's legs. In one setting, the pretension in the exotendon can be in a range of about 4.5kg (10 lbs) to 13.6kg (30 lbs), and can be increased or decreased at any time. A ratio of a minimum tensile force on the exotendon to a maximum tensile force on the exotendon can be in a range of about 3 to 20 during, for example, normal walking. The maximum tensile force on the exotendon can be in the range of about 2.1 kg-force meter (15 lb_{f}) to about 8.3kg-force meter (60 lb_{f}). The minimum tensile force on the exotendon can be less than about 2.8kg-force meter (20 lb_{f}), 2.1 kg-force meter (15 lb_{f}), or 1.4kg-force meter (10 lb_{f}) during normal walking.

In some embodiments, a system for assisting body movement of a user comprises right and left orthosis apparatuses. The right orthosis apparatus is wearable on the right leg of the user. The right orthosis apparatus stores energy as the right leg moves posteriorly relative to the user's torso and releases stored energy to help move the right leg. The left orthosis apparatus is wearable on the left leg of the user and stores energy independently of the right orthosis apparatus. The left orthosis apparatus is configured to store energy as the left leg moves posteriorly relative to the user's torso and releases stored energy to assist movement of the left leg.

One or both of the right and left orthosis apparatuses can generate a moment proximate to the user's hip to ensure that the orthosis comfortably helps rotate the user's legs. The right orthosis apparatus, in some embodiments, includes a right exotendon that stores energy as the right leg moves rearwardly and releases the stored energy to help move the right leg forwardly. The left orthosis apparatus includes a left exotendon capable of storing energy independent of operation of the right exotendon. The left exotendon stores energy as the left leg moves rearwardly relative to the user's torso and releases stored energy to help move the leg.

In some embodiments, an orthosis includes a belt assembly configured to be secured to the user's body. The belt assembly can include a first arm having a first mounting end, a second arm having a second mounting end, and a back portion. The back portion can be coupled to a first mounting end of a first arm and the second mounting end of the second arm. The back portion can have at least one positioner extending vertically along the user's back when the first arm and the second arm wrap around the user's body (e.g., waist, pelvis, hips, or the like). A leg frame assembly is wearable on the leg of the user and is coupled to the belt assembly.

The positioner can include an anatomical feature locator corresponding in shape to an anatomical feature of the user's body such that the anatomical feature locator engages the anatomical feature of the user's body to align the belt assembly. In some embodiments, the anatomical feature locator is a protrusion or a recess configured to preferentially seat against the user's body.

In some embodiments, the back portion includes a stiffener (e.g., a rigid plate) that extends vertically from the first and second mounting ends along the user's back. For example, the stiffener can protrude upwardly from the arms and overly a portion of the user's back. When a force from the leg assembly is applied to the belt assembly, the belt assembly can maintain its shape to ensure that the leg assembly remains properly aligned with the user's body.

The belt assembly, in some embodiments, includes a belt main body and padding. The belt main body can be made of material that is less compliant than the padding. In some embodiments, the belt main body is made of rigid plastic, metal, or other material capable of withstanding relatively high tensile loads. The padding can be made, in whole or in part, of open-cell foam, closed-cell foam, or the like. The padding can include a cover that surrounds a cushioning material. In some embodiments, the padding is attached to the main body by one or more fasteners (e.g., hook and loop type fastener), snaps, ties, or the like. The padding can be removed from the belt main body for cleaning or maintenance.

### BRIEF DESCRIPTION OF THE DRAWINGS

Many aspects of the present disclosure can be better understood with reference to the following drawings. Identical reference numbers identify similar elements or acts.
Figure 1 is an isometric view of an orthosis in accordance with an embodiment of the present technology.
Figure 2 is an isometric view of a belt assembly in accordance with an embodiment of the present technology.
Figure 3 is an isometric view of a foot ankle assembly in accordance with an embodiment of the present technology.
Figure 4 is an isometric view of an orthosis in accordance with an embodiment of the present technology.
Figure 5 is a side elevational view of the orthosis of Figure 4.
Figure 6 is a front elevational view of the orthosis of Figure 4.
Figure 7 is an exploded isometric view of the orthosis of Figure 4.
Figure 8 is a side elevational view of an adjustment mechanism in a locked state in accordance with an embodiment of the present technology.
Figure 9 is a side elevational view of an adjustment mechanism in an unlocked state in accordance with an embodiment of the present technology.
Figure 10 is an exploded isometric view of an adjustment mechanism in accordance with an embodiment of the present technology.
Figure 11 is an isometric view of a belt assembly in accordance with an embodiment of the present technology.
Figure 12 is a side elevational view of the belt assembly of Figure 11.
Figure 13 is a front elevational view of the belt assembly of Figure 11.
Figure 14 is a plot of time versus applied forces in accordance with some embodiments of the present technology.
Figure 15 is a plot of time versus applied force in accordance with some embodiments of the present technology.
Figure 16A is a plot of a gait cycle versus applied force.
Figure 16B is a plot of a gait cycle versus joint moments.
Figure 17 is an isometric view of a motorized orthosis in accordance with an embodiment of the present technology

### DETAILED DESCRIPTION

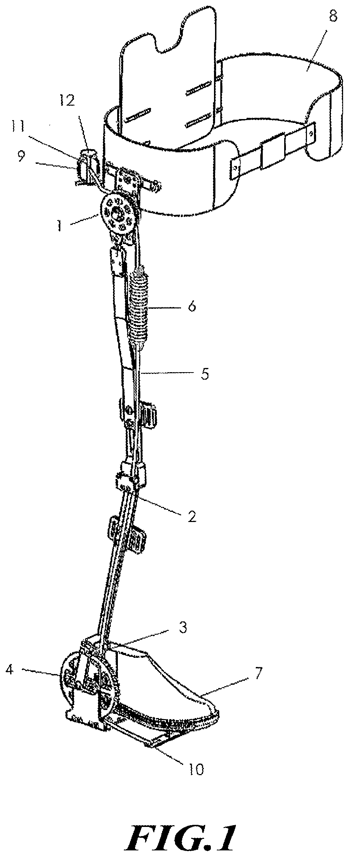

Figure 1 is an isometric view of an orthosis in the form of a hip-knee-ankle-foot orthosis that includes four pulleys 1, 2, 3, 4 and an extendable exotendon 5. The exotendon 5 includes cables and a spring 6. The exotendon 5 includes a spring 6 and wraps around the pulleys 1, 2, 3, 4. One end of the exotendon 5 is coupled to a shoe 7 and another end of the exotendon 5 is coupled to a hip belt 8. A mechanism 9, illustrated as a ratcheting/spooling mechanism, is configured to adjust the tension of the exotendon 5.

The exotendon 5 can include cables made, in whole or in part, of one or more polymers, plastics, carbon (*e.g*., carbon fibers), nylon, braided webbing, rope, cables, or some other non-metal or metal material. Metal exotendons can include one or more metal segments that are connected by one more springs (e.g., helical springs, tension springs, or the like) made, in whole or in part, of metal, rubber, polymers, or the like. The springs can have linear behavior consistent with Hooke's law. The exotendon 5 is capable of storing energy produced by movement of the leg and is capable of releasing the stored energy to help the user move the leg. In some embodiments, the tension force applied to the spring 6 increases as the user pushes his/her body forward. The spring 6 stores energy until the user pushes off of the ground. The spring 6 releases energy by contracting so as to help move the user's leg forward.

Each of the pulleys 1, 2, 3, 4 can include a round disk, a semicircular disk, a partial disk, or a lever arm. The pulleys 1, 2, 3, 4 can be rotatably coupled to the frame. The exotendon 5 of Figure 1 terminates at a termination region 10 coupled (*e.g.,* fixedly coupled) to a sole of footwear 7 (*e.g*., a shoe, a boot, etc.) and mounted underneath the user's foot. The illustrated region 10 is positioned underneath the ball of the user's foot. The exotendon 5 leaves the termination region 10 and travels rearwardly, substantially parallel to the sole of the footwear 7 towards the bottom of the ankle pulley 4. The ankle pulley 4 can be located off axis and above the ankle joint center such that the bottom of the pulley is adjacent the ground. In some embodiments, the bottom of the pulley 4 is positioned as close to the ground as possible. The exotendon 5 can thus extend generally parallel to the ground and between the ankle pulley 4 and the termination region 10. The exotendon 5 can be at other locations and orientations, if needed or desired.

The exotendon 5 wraps around the backside of the ankle pulley 4 and over the front side of the calf pulley 3 before continuing on to the knee pulley 2. The calf pulley 3 can be mounted to a calf bracing or frame material member than connects the ankle and knee joints. The calf pulley 3 can be located immediately above the ankle pulley 4 or at any other suitable location.

The exotendon 5 can extend away from the calf pulley 3 and travel across the front side of the knee pulley 2 and connect to the spring 6 between the knee and the hip joints. The exotendon 5 leaves the spring 6 and runs over the top of the hip pulley 1. The hip pulley 1 can be located off axis and above the operator's hip joint center or at any other suitable location.

After wrapping over at least a portion of the hip pulley 1, the exotendon 5 terminates at a termination region 11 coupled to the belt. The exotendon 5 can be rigidly coupled to the belt 8 at a location behind and at the same general height as the top of the hip pulley 1. The termination region 11 can include a spool which allows slack in the exotendon 5 to be removed by turning a knob 12 and spooling the excess cables onto the center shaft 11. A ratchet mechanism can prevent unspooling of the exotendon 5 and allows the operator to apply a preload. The preload can be selected based on the desired operation by the user. In some embodiments, the preload is in a range of about between 0 and 22.7kg (zero and fifty pounds). Other preloads are also possible, if needed or desired. A button can disengage the ratchet and allow the tension in the exotendon 5 to be released by unspooling of the exotendon 5.

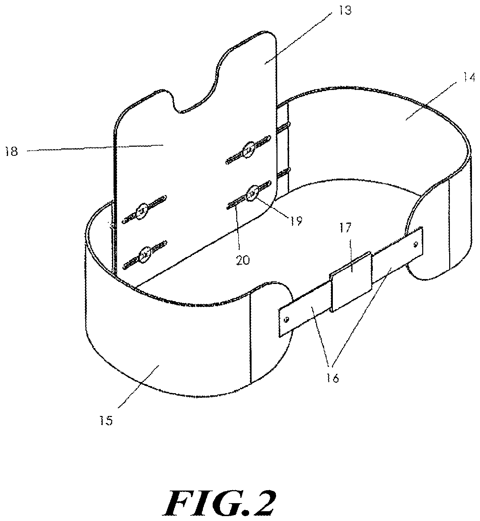

Figure 2 is an isometric view of the belt comprised of a back member 13, two arms 14 and 15, webbing 16, and a front buckle 17. Padding lines the interior of the belt and is not shown for clarity. The belt can have a rise in the back 18 for resisting belt rotation by pushing against the operator's back as the exotendons become loaded. Advantageously, adjustments to the belt length can be made without changing the location of the hip pulleys relative to the operator's hip joints. In some embodiments, adjustments to belt length can be made in the front via the buckle 17 and webbing 16. Arms 14 and 15 are coupled to the back member 13 via fasteners. The illustrated fasteners are screws and t-nuts 19 which pass through aligned slots 20 cut out of the back 13 and arms 14, 15. Adjustments can be made to the belt diameter in the rear by loosening the screws and t-nuts 19, sliding the arms 14 and 15 into the appropriate position and then retightening the fasteners 19. Other types of belts can also be used and can include different types of fasteners, stiffeners, positioning members, elastic members, straps, or the like suitable for coupling an orthosis to a user's body.

In non-limiting embodiments, hip pulleys can range in size from about one and a half inches to about 12.7cm (five inches) in diameter, while ankle pulleys range in size from about 10.2cm (four inches) to about 30.5cm (twelve inches) in diameter. In some embodiments, the hip pulley has a diameter of about 15.2cm (6 inches). The stiffness of the spring 6 can be in the range of about 0.2kg per cm (one pound per inch) to 5.4kg per cm (thirty pounds per inch), and the displacement of the spring throughout one gait cycle is between 3.8 and 10.2cm (one half and four inches). Other dimensions, stiffnesses, and spring displacements are also possible, if needed or desired. The exotendon can include any number of separate springs or integral springs.

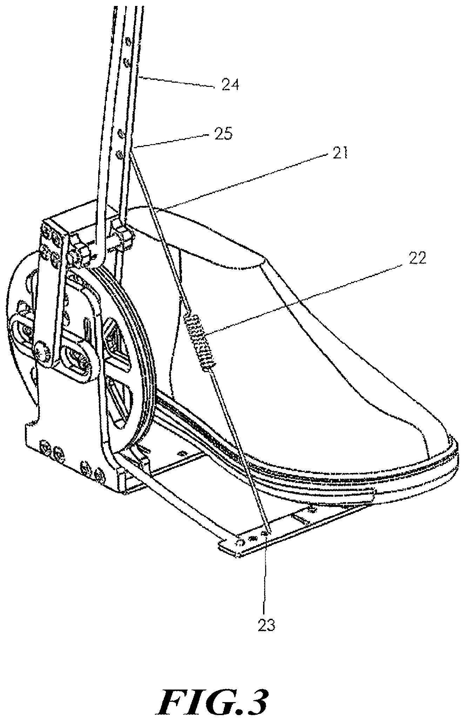

Figure 3 is an isometric view of a foot ankle assembly. A cable 21 and a spring 22 oppose the exotendon 5 and attach to the shoe 7 at a foot tie off point 23 and to the calf bracing 24 at the calf tie off point 25. The spring 23 can help counter foot drop. The stiffness, initial tension, and preload of the spring 22 during stance can be selected such that it counteracts foot drop during the swing phase of gait, while not significantly counteracting the energy storage in the spring 6 during the stance phase of gait.

The exotendon 5 terminates at the toe termination region 10 and wraps behind the ankle pulley 4, in front of the calf pulley 3, knee pulley 2 and hip pulley 1, and terminates at the hip termination mechanism 9 mounted to the belt 8 behind and in line with the top of the ankle pulley 1. In use, preload tension is added to the exotendon 5 during stance, such that between about 0 to about 22.7kg of force (zero to about fifty pounds of force) is applied to the exotendon 5 during stance. Tension is applied by turning the knob 12 connected to the belt termination region 11 (Figure 1), which spools exotendon 5 onto the center shaft 11. The ratchet mechanism prevents the exotendon 5 from unspooling. Throughout the stance phase of gate, the distance the exotendon 5 travels between the belt 8 and the foot increases. This displacement stretches the spring 6 and energy is stored. During the swing phase of the gait the energy stored in the spring 6 is returned to the system and
provides assistance for walking, running, etc. The high back 18 on the belt 8 resists belt rotation when the exotendon 5 is tensioned.

The belt diameter and fit can be adjusted in the front and the back so that the hip pulleys 1 remain generally fixed relative to the operator's hip joint centers as the belt 8 is tightened. The belt 8 can be tightened in the front by pulling webbing 16 through the center buckle 17. The belt 8 can be tightened in the back by loosening the screws and t-nuts 19 that bind the back member 13 to the arms 14 and 15, and sliding the arms 14 and 15 relative to the back member 13. Once the arms 14 and 15 are in the desired position relative to the back member 13, the screws and t-nuts 19 are retightened, thereby holding the back member 13 and the arms 14 and 15 together.

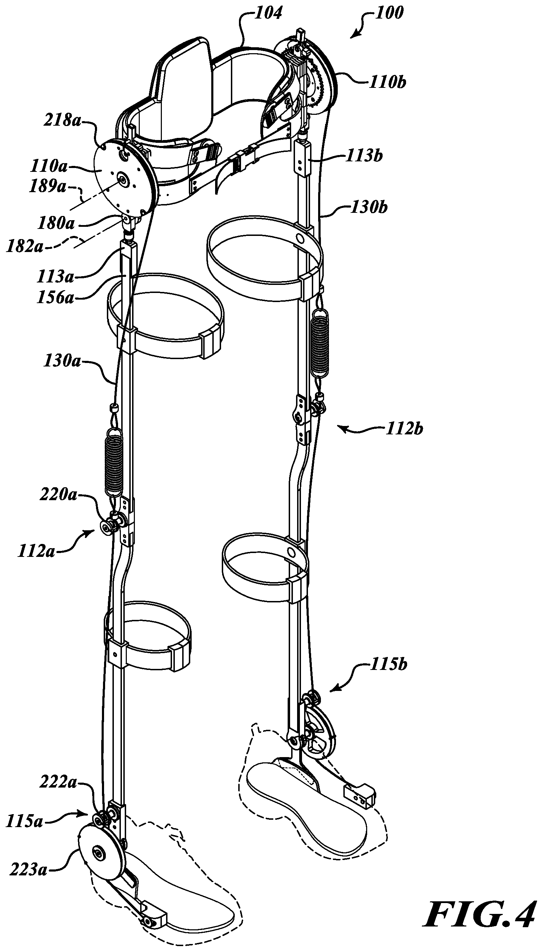

Figure 4 shows an orthosis 100 in accordance with at least some embodiments of the present technology. The orthosis 100 is symmetrical with respect to the median plane of the user and the description of a component on one side of the median plane applies equally to the corresponding component on the other side of the median plane, unless clearly indicated otherwise.

The orthosis 100 includes a belt assembly 104, energy storage apparatuses 110a, 110b (collectively "110"), and leg frame assemblies 112a, 112b (collectively "112"). The belt assembly 104 can securely hold the user to position the leg frame assemblies 112a, 112b alongside the user's right and left legs, respectively. The leg frame assemblies 112a, 112b have upper ends 113a, 113b and lower portions 115a, 115b. The upper ends 113a, 113b are rotatably coupled to the belt assembly 104. The lower portions 115a, 115b are coupleable to lower portions of a user's right and left legs, respectively. Each exotendon 130a, 130b stores energy as the leg to which it is coupled moves posteriorly and releases the stored energy to help move the leg forward. Significant amounts of energy can be repeatedly stored and released to assist body movement of the user.

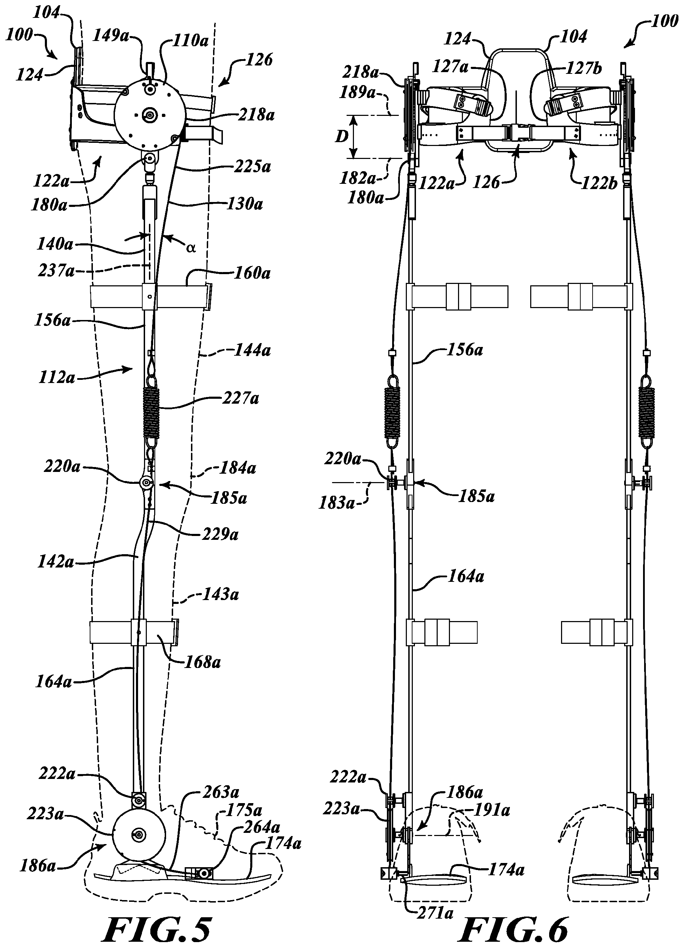

Referring to Figures 5 and 6, the belt assembly 104 can include a pair of arms 122a, 122b (collectively "122"), a back portion 124, and a restraining system 126. The arms 122 can surround at least a portion of the user's waist, hips, pelvic torso, and/or other anatomical structure. The back portion 124 extends upwardly and can help minimize, limit, or substantially eliminate rotation of the arms 122 relative to the user's body. The restraining system 126 can couple together arm ends 127a, 127b (Figure 6). When the restraining system 126 is in the closed configuration, the belt assembly 104 can surround the user's body. The restraining system 126 can be opened to move or adjust the position of the belt assembly 104. In some embodiments, the ends 127a, 127b are coupled together by one or more belts, snaps, hook and loop type fastener (e.g., VELCRO® brand fasteners), lacing, or the like.

The description of one energy storage apparatus 110a, 110b applies equally to the other energy apparatus, unless clearly indicated otherwise. The energy storage apparatus 110a of Figure 5 is coupled to the belt assembly 104 and the leg frame assembly 112a. The energy storage device 110a in a locked state can hold the extended or stretched exotendon 130a and in an unlocked state allows contraction of the exotendon 130a. When the user stands without any knee flexion, the exotendon 130a can be pretensioned, if desired. The amount of preload force can be selectively increased or decreased using an adjustment mechanism 149a. In some embodiments, the pretension force in the exotendon 130a can be in a range of about 4.5kg (10 lbs) to about 22.7kg (50 lbs). Other pretension forces can be applied, if needed or desired.

Referring to Figure 5, the leg frame assembly 112a has an upper portion 140a rotatably coupled to the energy storage apparatus 110 and a lower portion 142a coupleable to a lower portion 143a of a user's leg 144a (shown in a phantom line). An upper frame member 156a can be coupled to the femoral portion of the leg 144a by a leg holder 160a (e.g., a band, a strap, a belt, or the like). The upper frame member 156a can be made, in whole in part, of metal (e.g., aluminium, steel, or the like), a composite material (e.g., carbon fiber reinforced composite), and/or plastics formed by an extrusion process, molding process, machining process, or other suitable process for forming a member that can bear a significant load without significant deformation. A lower frame member 164a extends downwardly and can be coupled to a tibial portion of the leg 144a by a leg holder 168a.

The user's foot can be on a foot platform 174a which can be configured to fit inside of footwear 175a (e.g., a shoe, a boot, or the like). In some embodiments, the foot platform 174a is a foot orthosis upon which the user's foot can be placed and can be made, in whole or in part, of a rigid plastic, metal, a composite material, or other materials capable of retaining its shape during use. The foot platform 174a can include a cushioning layer made of a compliant material (e.g., rubber, foam, or the like). For example, the foot platform 174a can include a lower rigid layer and an upper cushioning layer adhered to an upper surface of the lower rigid layer. When the exotendon 130a is tensioned, the foot platform 174a can maintain its shape to limit or prevent bending of the user's foot. In some embodiments, the platform 174a and the footwear 175a are integrated together. For example, the footwear 175a can be a shoe with a rigid sole configured to prevent unwanted deflection of the user's foot. One or more stops (e.g., hard stops) can be used to prevent or limit foot drop. The stops can be protrusions or other stationary features that physically contact and limit movement of the foot platform 174a. Alternatively, one or more biasing members (e.g., springs) can be used to control foot location.

Referring again to Figures 4 and 6, a hip frame joint 180a couples the belt assembly 104 to the upper frame member 156a. The hip frame joint 180a allows the upper frame member 156a to rotate about an axis of rotation 182a. The axis of rotation 182a can be generally aligned with a pivot axis of the user's right hip joint can be generally parallel to or disposed in a coronal plane dividing the body into equal front and back parts. The axis of rotation 182a can also be generally perpendicular to the median plane which divides the user's body into equal right and left halves. The position and orientation of the axis of rotation 182a can be adjusted to provide a comfortable fit.

Referring to Figure 6, the axis of rotation 189a of the energy storage apparatus 110a is spaced apart from the axis of rotation 182a. A distance D between the axes of rotation 182a, 189a can be equal to or greater than about 2.5cm, 5.1 cm, 7.6cm, 12.7cm, 17.8cm (1 inch, 2 inches, 3 inches, 5 inches, 7 inches), or the like. Other distances are also possible, if needed or desired.

Referring to Figures 5 and 6, a knee frame joint 185a can rotatably couple the upper frame member 156a to the lower frame member 164a and can be positioned adjacent to a knee 184a of the right leg 144a. The knee frame joint 185a can define an axis of rotation 183a about which the upper frame member 156a and lower frame member 164a can rotate. The axis of rotation 183a can be generally aligned with a pivot axis of the knee 184a and can extend generally parallel to the hip axis of rotation 182a. In some embodiments, the knee frame joint 185a comprises a mechanical stance control knee joint or electromechanical stance control knee joint capable of providing a desired amount of knee flexion contracture. The knee frame joint 185a can allow locking and unlocking based on, for example, the stance phase.

An ankle frame joint 186a of Figure 6 rotatably couples the lower frame member 164a to the platform 174a. The ankle frame joint 186a defines an ankle axis of rotation 191a which is disposed in general alignment with a pivot axis for an ankle of the leg 144a. The ankle axis of rotation 191a can extend generally parallel to the hip axis of rotation 183a.

Figures 4-6 show rotatable members 218a, 220a, 222a, 223a. When the right leg is bent in flexion, rotatable members 218a, 220a, 222a, 223a cooperate to apply a tensile force to the exotendon 130a. The rotatable members 218a, 220a, 222a, 223a can be spools, guide wheels, arcuate members, or the like. For example, the rotatable member 218a can be a spool about which the exotendon 130a can be wrapped, and the rotatable member 222a can be a guide wheel.

The exotendon 130a can resiliently stretch and store potential energy. When the right leg is positioned rearwardly, the energy stored in the exotendon 130a can be released as the exotendon 130a contracts to help drive the leg forward. In some embodiments, the exotendon 130a helps begin leg swing. As shown in Figure 5, the exotendon 130a extends alongside the frame assembly 112.

The exotendon 130a can extend along most of a length of the user's leg and can store most of the energy in a biasing member in the form of a tension spring 227a. The length, position, and orientation of the exotendon 130a can be selected to achieve desired action. As shown in Figure 5, an angle α is defined by a longitudinal axis 237a of the upper frame member 156a (or femur) and a portion of the exotendon 130a. The angle α can be increased or decreased to increase or decrease the rate of stretching of the exotendon 130a and can be equal to or greater than about 5 degrees, 10 degrees, 15 degrees, 20 degrees, or 30 degrees. Other angles are also possible, if needed or desired.

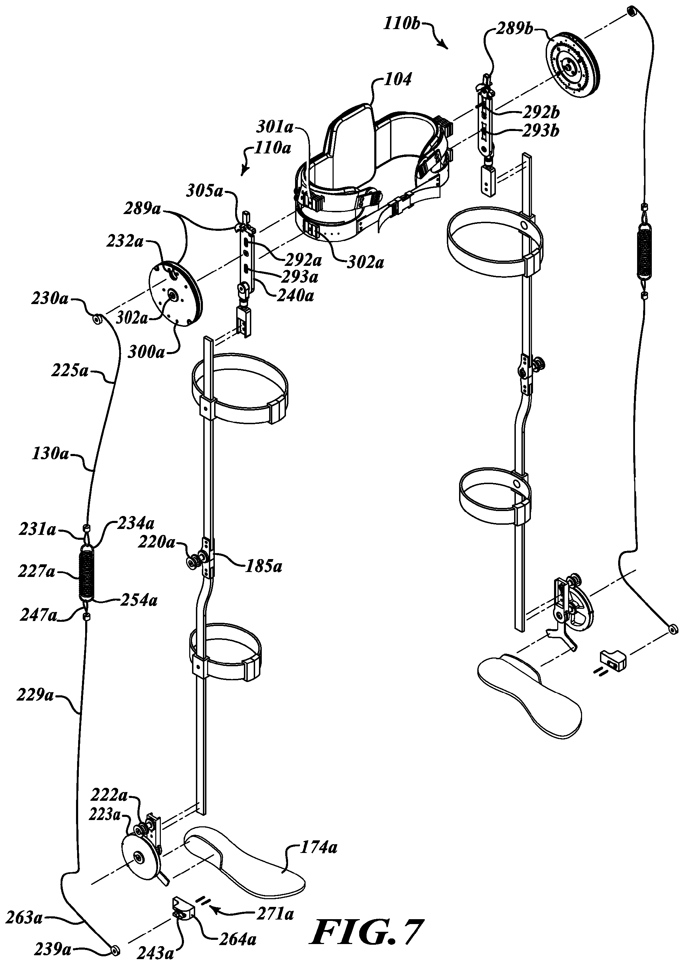

As shown in Figure 7, the exotendon 130a can include an upper cable 225a, the spring 227a, and a lower cable 229a. The upper cable 225a can include a coupler 230a (e.g., a disk-shaped member, a retainer, or the like) receivable by a receiving opening 232a and a connector end 231a (illustrated as a loop) coupled to an upper end 234a of the spring 227a.

The spring 227a can be positioned adjacent to the upper frame member 156a and can include one or more tension springs (illustrated), helical springs, elongatable members (e.g., rubber elongate members), bands, or the like. The spring 227a can be made of metal, polymers, elastomers, combinations thereof, or the like. The illustrated spring 227a is a tension spring made, in whole or in part, of steel (e.g., spring steel). In some embodiments, a plurality of springs can be coupled together.

Referring to Figure 7, the lower cable 229a includes a coupler 239a receivable by a receiving opening 243a of a foot coupler 264a and a connector end 247a (illustrated as a loop) coupled to a lower end 254a of the spring 227a. The lower cable 229a extends downwardly from the spring 227a through the rotatable member 220a (Figure 5). The rotatable member 220a can be a guide wheel rotatably coupled to the knee joint 185a. The lower cable 229a extends downwardly from the member 220a to the rotatable member 222a, illustrated in the form of a guide wheel positioned generally above the rotatable member 223a. The lower cable 229a extends in front of the guide wheel 222a and between the guide wheel 222a and the rotatable member 223a. The lower cable 229a wraps around the backside of the rotatable member 223a. A portion 263a of the lower cable 229a extends past the rotatable member 223a to the foot coupler 264a.

In some embodiments, the coupler 264a can be coupled to the exterior of the user's footwear 175a (Figures 4-6) by one or more fasteners 271 a. The fasteners 271 a can be coupled to the platform 174a. In other embodiments, the foot coupler 264a can be part of the footwear. In yet other embodiments, the coupler 264a can be located within the footwear. In some embodiments, the cable 229a can be incorporated into the footwear.

Referring to Figures 5-7, the vertically extending portion of the exotendon 130a is positioned alongside the leg. In other embodiments, the exotendon is positioned in front of the leg. For example, the exotendon can be positioned along the front of the leg (e.g., proximate to the thigh, knee, and shin). In other embodiments, the exotendon is positioned in back of the leg and can be positioned adjacent to the hamstring, knee, calf, and the heel. The exotendon can also be at other locations.

Figure 7 shows the energy storage apparatus 110a that includes an adjustment mechanism in the form of a pretension adjustment mechanism 289a and a mounting bracket 290a. Fasteners (e.g., screws, bolts, or the like) can pass through slots 292a, 293a and into brackets 301 a, 302a, respectively, to couple both the adjustment mechanism 289a and mounting bracket 290a to the belt assembly 104. The illustrated adjustment mechanism 289a functions as a ratchet and includes a pawl 305a and a spool 300a. The pawl 305a is positioned at the top of the bracket 290a, and the spool 300a is rotatably coupled to the bracket 290a by a pivot 302a.

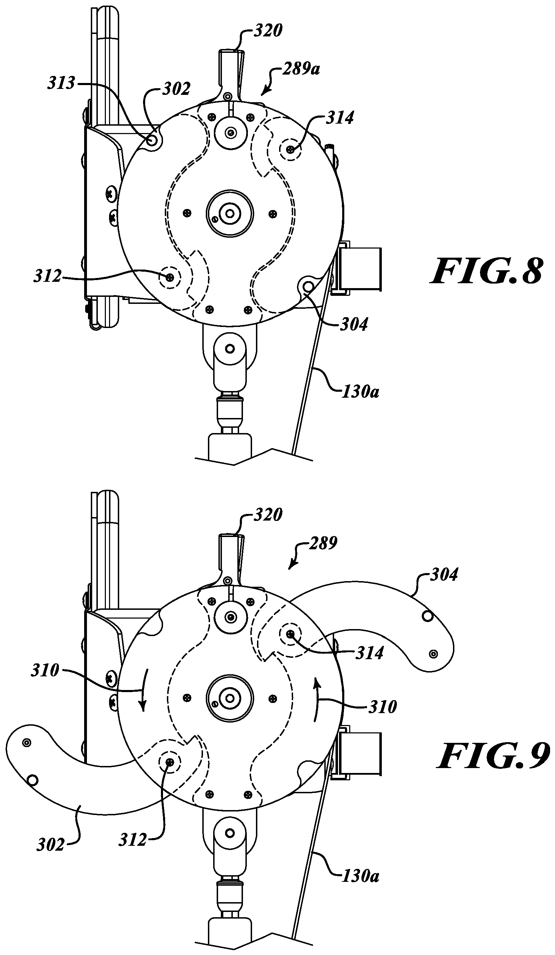

Figure 8 shows the adjustment mechanism 289a in a locked configuration. Figure 9 shows the adjustment mechanism 289a in an unlocked configuration. The adjustment mechanism 289a includes deployable arms 302, 304 movable between hidden positions (Figure 8) to exposed positions (Figure 9). To adjust the tension in the exotendon 130a, the arms 302, 304 are rotated about pivots 312, 314. A protrusion 313 can be used to move the arm 302 from the hidden position to the exposed position. In some embodiments, the arm 302 can be rotated about the pivot 312 an angle of about 70 degrees to 110 degrees. For example, the arm 302 of Figures 8 and 9 can be rotated about 90 degrees about the pivot 312.

Referring to Figure 9, the user can manually grip the arms 302, 304 to torque the adjustment mechanism 289a, as indicated by arrows 310. As the adjustment mechanism 289a rotates in the counterclockwise direction, the forced applied to the exotendon 130a increases and causes elongation or stretching of the exotendon 130a. After adjustment, the arms 302, 304 can be returned to their hidden positions.

A user can periodically adjust the preload force, if any, in the exotendon 130a based upon, for example, the activity to be performed. If a user is fatigued or weak, the tension of the exotendon 130a can be increased to provide an increased amount of assistance with body movement. To reduce assistance, the tension in the exotendon 130a can be decreased.

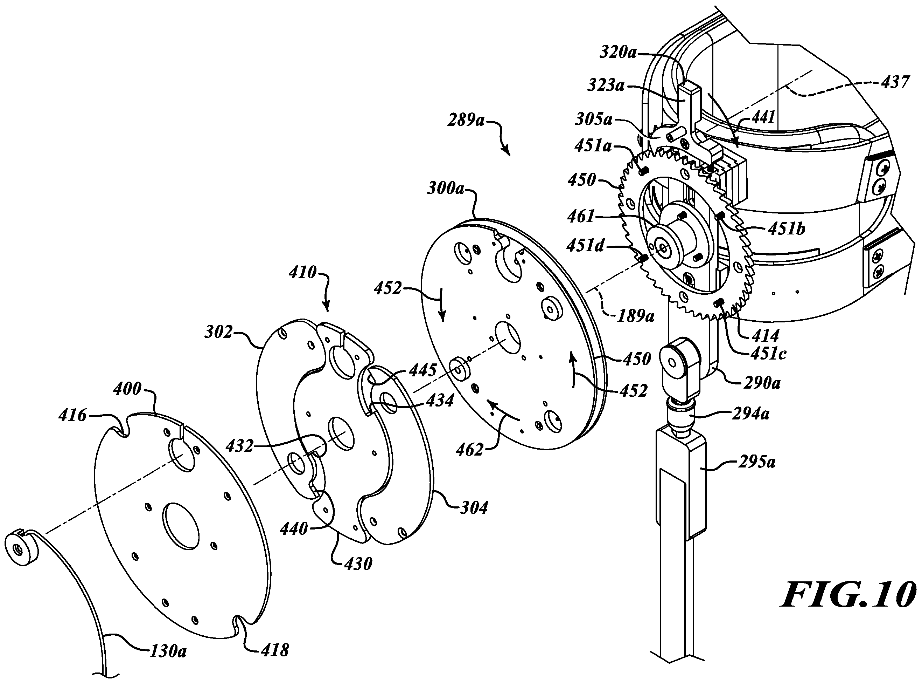

Figure 10 is an exploded isometric view of the adjustment mechanism in accordance with some embodiments of the present technology. A release device 320a is operable to unlock the adjustment mechanism 289a and allow rotation of the spool mechanism 300 in the clockwise direction to unspool the exotendon 130a. The release device 320a includes a lever arm 323a and the pawl 305a. The release device 320a can have other configurations. In some embodiments, the release device 320a includes a push button for operating a one-way ratchet mechanism.

The adjustment mechanism 289a further includes a cover 400, an arm assembly 410, the spool 300a, and a gear assembly 414. The cover 400 can be coupled to the spool 300a to cover the arm assembly 410. In some embodiments, including the illustrated embodiment of Figure 10, the cover 400 includes recesses 416, 418 that provide access to the arms 302, 304.

The arm assembly 410 includes the arms 302, 304 and a central member 430. The central member 430 has central stops 432, 434 for limiting rotation of the arms 302, 304, respectively. The arm 302 can rotate until an arm stop 440 contacts the central stop 432. The arm 304 can rotate until an arm stop 445 contacts the central stop 434.

A spool 300a is rotatably coupled to the bracket 290a by a pivot member 461. The spool 300a has a groove 450 capable of receiving the exotendon 130a. The exotendon 130a can wrap around the spool 300a for convenient storage. The spool 300a can be made, in whole or in part, of metal, plastic, or polymers, and can have an outer diameter in a range of about 10.2cm (4 inches) to about 20.3cm (8 inches). Other diameters can also be used based on the desired amount of adjustability.

The gear assembly 414 can be fixedly coupled to the spool 300a by a plurality of fasteners 451 a, 451 b, 451 c, 451 d such that the gear assembly 414 and spool 300a rotate together. When a user rotates the spool 300a in the counterclockwise direction (indicated by arrows 452), the pawl 305a moves in and out of the teeth located at the outer periphery of the gear assembly 414. The teeth can be inclined to allow counterclockwise rotation of the gear assembly 414 while the pawl 305a slides into and out of the teeth. The pawl 305a prevents rotation of the gear assembly 414 in the opposite direction. When the exotendon 130a applies a moment (represented by arrow 462) in the clockwise direction, the pawl 305a can prevent rotation of the gear assembly 414.

The lever arm 323a of the release device 320a can be rotated about an axis of rotation 437 (e.g., rotated in a clockwise direction as indicated by an arrow 441) to move the pawl 305a away from the gear assembly 414. After the pawl 305a is spaced apart from the teeth, the gear assembly 414 can freely rotate in the clockwise direction about the axis of rotation 189a to reduce the tension, if any, in the exotendon 130a. Other types of puller units (e.g., electromechanical adjustment mechanisms), one-way ratchet mechanisms, tensioners, and locking mechanisms can also be used.

Figure 10 shows a connector 295a rotatably coupled to the bracket 290a by joint 294a. The joint 294a can permit internal rotation and/or external hip rotation. In some embodiments, the joint 294a can allow internal hip rotation in a range of about 40 degrees to about 80 degrees and can allow external hip rotation in a range of about 30 degrees to about 60 degrees. In some embodiments, the hip rotation can be limited or minimized for increased stability. In some embodiments, the joint 294a can be in the form of a ball and socket joint or other type of joint that provides at least two degrees of freedom. In other embodiments, the joint 294a can include a plurality of pivots, each allowing rotation about a different axis of rotation.

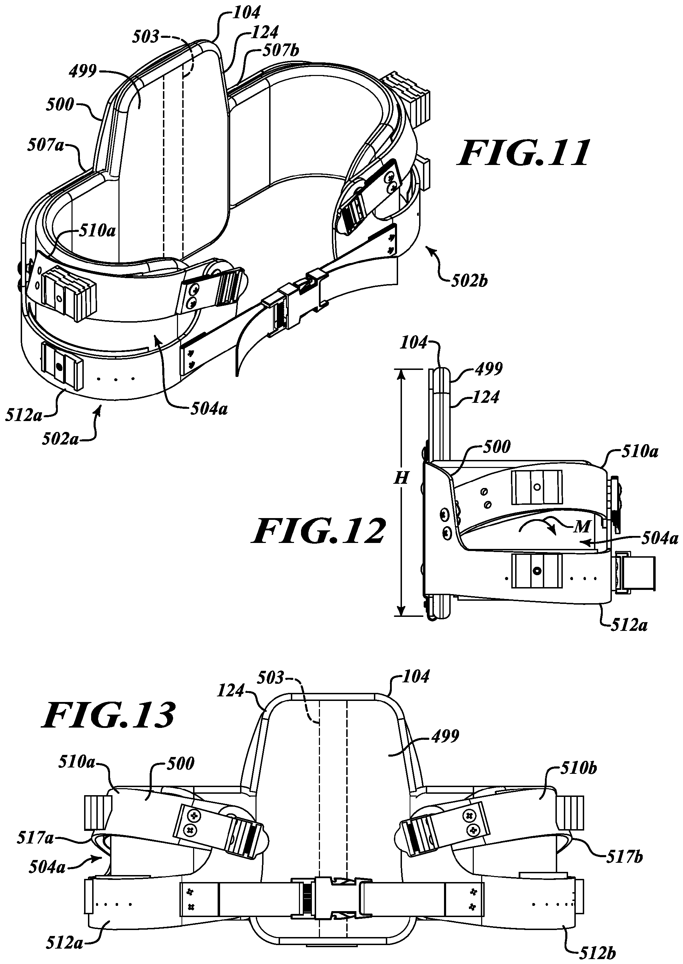

Referring to Figures 11-13, the belt assembly 104 includes padding 499 and a main body 500. The padding 499 can include a cushioning material and a covering (e.g., an air permeable layer, high wear material, or the like) surrounding the cushioning material. The cushioning material can be open-cell foam, closed-cell foam, or the like and can be made, in whole or in part, of polyurethane, a memory material (e.g., viscoelastic polyurethane.), or other highly compressible material than can comfortably surround the user. The padding 499 can be detachably coupled to the main body 500 by hook and loop type fasteners, snaps, etc.

The back portion 124 can have one or more alignment features configured to keep the belt assembly 104 positioned relative to the user's body. Non-limiting exemplary alignment features 503 (illustrated in phantom line) can include, without limitation, one or more ribs, grooves, protrusions, or the like that can interact with the anatomical structures to reduce, limit, or substantially eliminate movement between the belt assembly 104 and the user body, thereby limiting or minimizing misalignment of the energy storage apparatuses 110a, 110b. In some embodiments, the alignment feature 503 is a vertically extending recess or protrusion positioned in the middle of the back portion 124. The alignment feature 503 can mate with the user's back or spine such that interaction between alignment feature 503 and the user's body can help maintain alignment of the belt assembly 104. In some embodiments, the alignment feature 503 is an elongate protrusion that can fit conveniently between portions of the sacrospinalis on either side of the spinous process. The belt assembly 104 can have other types of anatomical alignment features corresponding in shape to an anatomical feature of the user.

Referring to Figure 11, the main body 500 includes arm assemblies 502a, 502b (collectively, "502") with mounting ends 507a, 507b coupled to the back portion 124. The arm assembly 502a can include an upper portion 510a and a lower portion 512a that are spaced apart to define a window 504a. A portion of the user's hip, or pelvis can be received in the window 504a. As shown in Figure 13, the upper portions 510a, 510b can define a narrower opening than an opening defined by the lower portions 512a, 512b. The lower edges 517a, 517b can comfortably rest on the user's hip or pelvis.

The back portion 124 of Figure 12 can have a height H equal to or greater than about 7.6cm, 12.7cm, 17.8cm, 22.9cm, 30.5cm (3 inches, 5 inches, 7 inches, 9 inches, 12 inches), or ranges encompassing such heights. As the leg frame assembly 112a moves rearwardly, a moment M (illustrated by arrow M of Figure 12) can be generated. The back portion 124 serves as a stiffener that prevents an appreciable amount of movement of the arm assemblies 502a, 502b. The height H and mechanical properties of the arm assemblies 502a, 502b can be selected to keep the other components of the orthosis 100 in the desired positions and orientations.

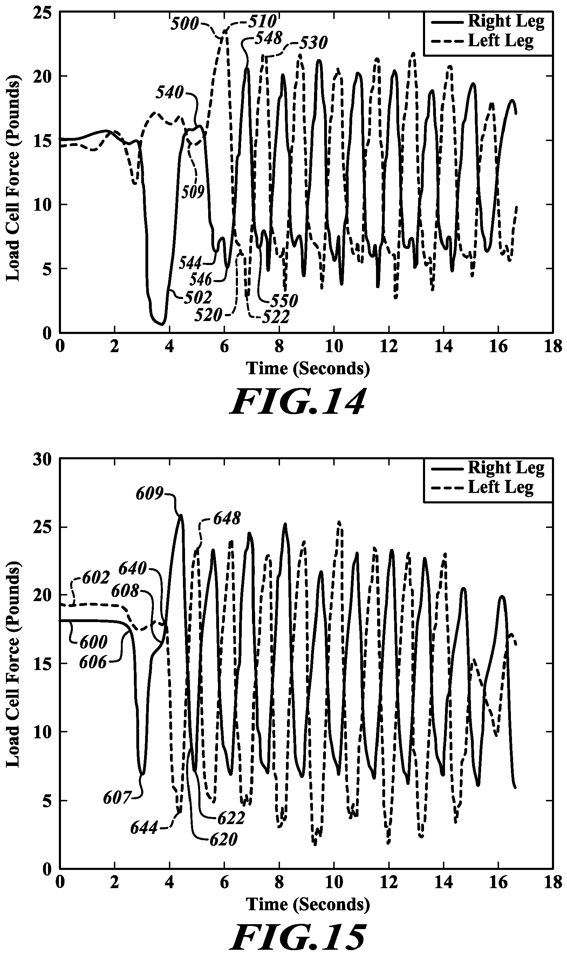

Figure 14 shows a plot of time versus applied force in accordance with at least some embodiments of the present technology. A curve 500 corresponds to the force applied to the exotendon 130b, and the curve 502 corresponds to the force applied to the exotendon 130a. A single gait cycle is shown from about T=5 seconds to about T=7 seconds. The exotendons 130a, 130b are pretensioned with a force of about 14 lbs to about 15 lbs. An adjustment mechanism (e.g., adjustment mechanisms 489a, 489b) can be used to selectively increase or decrease the pretension to adjust operation. If the user applies pretension force while sitting, the pretension force may be much higher when the user stands and the exotendons stretch more. Thus, the adjustments can be made when the user is standing.

From 0 to about 5 seconds, the user is preparing to walk. At 509, the user's body moves past the left leg and the load applied to the exotendon 130b increases. At 510, the user's leg is behind his/her torso as the left foot pushes off the ground. The load on the exotendon 130b is reduced from the local maxima of about 23.5 lbs at 510 as the user's leg swings forward. The energy stored in the elongated or stretched exotendon 130b is released as the exotedon 130b contracts to help the move the left leg forward. The user's left heel strikes the ground at 520. The force further decreases to a local minima at 522 corresponding to the user transferring his/her body weight on the left foot. The user's body moves over the left leg to increase the force of the exotendon 130a from 522 to 530.

At 540, the user's right leg is positioned generally underneath the user's body. The exotendon 130a is pretensioned with a force of about 6.8kg (15 lbs). As the right left moves forward, the force applied to the exotendon 130a decreases to a local minima of about 2.7kg (6
lbs) at 544 corresponding to when the user's heel strikes the ground. The user transfers weight corresponding to a local minima of 2.3kg (5 lbs) at 546. As the user moves his/her body forward and over the right foot, the force applied to the exotendon 130a increases as the right leg moves posteriorly of the user's torso. At 548, the user's right foot pushes off of the ground and the force applied to the exotendon 130a is about 9.5kg (21 lbs). The energy stored in the elongated or stretched exotendon 130a is released as the exotendon 130a contracts to help the move the right leg forward.

Figure 15 shows a plot of time versus force on an orthosis in accordance with at least some embodiments of the present technology. A curve 600 corresponds to the force applied to the exotendon 130a, and the curve 602 corresponds to the force applied to the exotendon 130b. The exotedons 130a, 130b are pretension with a force of about 7.7kg (17 lbs) to about 9.1kg (20 lbs). The energy storage apparatuses 110a, 110b can be operated independently to adjust the amount of pretension in the exotendons 130a, 130b independently. For example, the pretension in the exotendon 130a can be about 6.8kg (15 lbs) while the pretension of exotendon 130b is about 9.1 kg (20 lbs). Throughout the day, the user can independently adjust the amounts of pretension based on desired assistance with body movement. For example, if the wearer's right leg if fatigued, the user can increase the tension of the exotendon 130a without altering the settings of the energy storage apparatus 110b.

From 0 to about 2 seconds, the user is preparing to walk. A 606, the user's right leg is lifted and moves in front of the user. The exotendon 103a contracts and releases stored energy to help move the right leg forward as the force decreases from about 8.2kg (18 lbs) at 606 to about 2.7kg - 3.2kg (6 lbs - 7 lbs) at 607. From 607 to 608, user moves his/her body over the right leg and the exotendon 103 is stretched. From 608 to 609, the user's body moves past the right leg and the load applied to the exotendon 130a increases. At 609, the user's right leg is behind his/her torso as the right foot pushes off the ground. The load on the exotendon 130a is reduced from the local maxima of about 26 lbs at 609 as the user's right leg swings forward. The energy stored in the stretched exotendon 130a is released as the exotendon 130a contracts to help swing the right leg forward. The user's right heel strikes the ground at 620. The load on the exotendon 130a further
decreases to a local minima of about 2.7kg (6 lbs) to about 3.2kg (7 lbs) at 622 corresponding to the user transferring his/her body weight to the right foot.

The exotendon 130b is pretensioned with a force of about 17 lbs to about 20 lbs. At 640 of the curve 602, the user's left leg is positioned generally underneath his/her body. As the left leg moves forward, the force applied to the exotendon 130b decreases to a local minima of about 4 lbs at 644 corresponding to when the user's left foot is on the ground. As the user moves his/her body forward and over the left foot, the force applied to the exotendon 130b increases. From 644 to 648, the force applied to the exotendon 130b increases and causes elongation or stretching or the exotendon 130b. At 648, the user's left foot pushes off the ground and the force applied to the exotendon 130b is about 23 lbs to about 24 lbs. The energy stored in the stretched exotendon 130b is released as the exotendon 130b contracts to help propel the left leg forward.

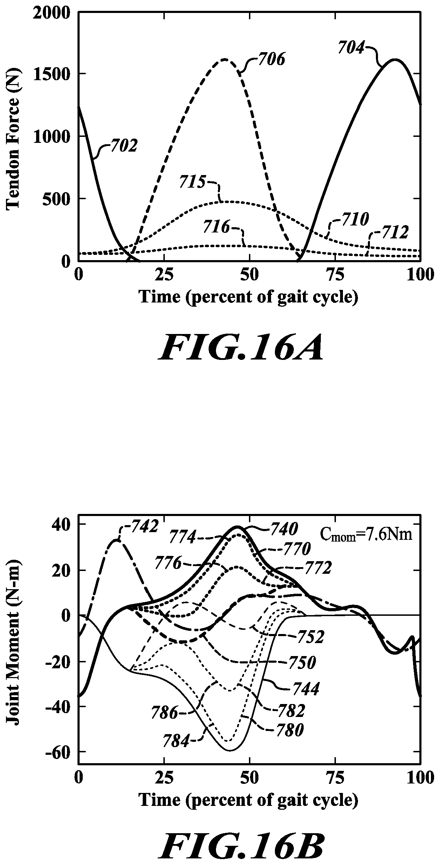

Figure 16A shows curves 702, 704 that correspond to the force applied to a spring in a left leg framework of a traditional exoskeleton (e.g., an exoskeleton similar to the exoskeleton disclosed in U.S. Patent No. 7,549,969). A curve 706 corresponds to the force applied to a spring of a right leg framework of a traditional exoskeleton. The springs are not pretensioned. For example, from 0% to about 15% of the gait cycle, the spring of the right leg framework is not tensioned while the right leg is in front or directly underneath the user's torso. The maximum applied force to the springs is relatively high (e.g., greater than 1,500 N) corresponding to when the user pushes off the ground. Such significant forces can be very uncomfortable and may not provide for desired movement.

In contrast, at least some of the embodiments of the present technology can provide relatively low forces, as shown in Figures 16A. The curves 710, 712 show an exemplary range of forces in the exotendons discussed in connection with Figures 1-13. The maximum curve 710 and minimum curve 712 can be produced using the orthosis 100 shown in Figure 4. At about 45% of the gait, there is a local maxima of about 500 N at 715. Another local maxima of about 100 N at 716 is on the curve 712. In some embodiments, a ratio of the maximum tendon force to minimum tendon force is in a range of about 2-10.

Figure 16B shows joint moments associated with a traditional exoskeleton and the joint moments of the orthosis 100 of Figure 4. Baseline curves 740, 742, 744 corresponding to moments at the hip, knee, and ankle, respectively, without the use of an orthosis. Curves 750, 752 correspond to moments about the hip and ankle, respectively, using a conventional orthosis (e.g., an exoskeleton similar to the exoskeleton disclosed in U.S. Patent No. 7,549,969).

The hip curves 770, 772 correspond to the maximum moment curve and minimum moment curve, respectively, of at least some embodiments of the orthosis 100 of Figure 4. Differences between the baseline hip curve 740 and the hip curves 770 or 772 correspond to the amount of work performed by the orthosis 100. A user has to perform more work if the orthosis 100 is set to generate the moments of the curve 770. The orthosis 100 provides assistance with hip joint from about 15%-20% to about 65% of the gait cycle. As shown in Figure 16B, the curves 770, 772 have generally the same shape as the curve 740. Thus, the characteristics of the moments generated by the user to, for example, walk are generally the same as the characteristics of the total moments of a natural gait.

At about 30% to about 35% of the gait, both curves 770, 772 gradually increase to local maximums 774, 776, respectively, at about 40% to about 50% of the cycle gait.

Ankle curves 780, 782 correspond to maximum and minimum curves of negative moments capable of being generated by the orthosis 100 of Figure 4. Differences between the ankle curves 780, 782 and the baseline ankle curve 740 correspond to the amount of work performed by the orthosis 100. The curves 780, 782 increase from a local maximum negative moment to a local minimum negative moment at about 25% to 30% of the gait cycle. The curves 780, 782 then increase to local maximum negative moments 783, 785 at about 40% to 50% of the gait cycle. The ankle joint moments decrease from the local maximum negative moments to local minimum negative moments at about 55% to 60% of the gait cycle. The curves 780, 782 have generally the same shape as the baseline ankle curve 744 such that the moments required to walk generally have the same profile as the forces required for natural movement. The embodiments disclosed herein thus provide for a relatively naturally gait.

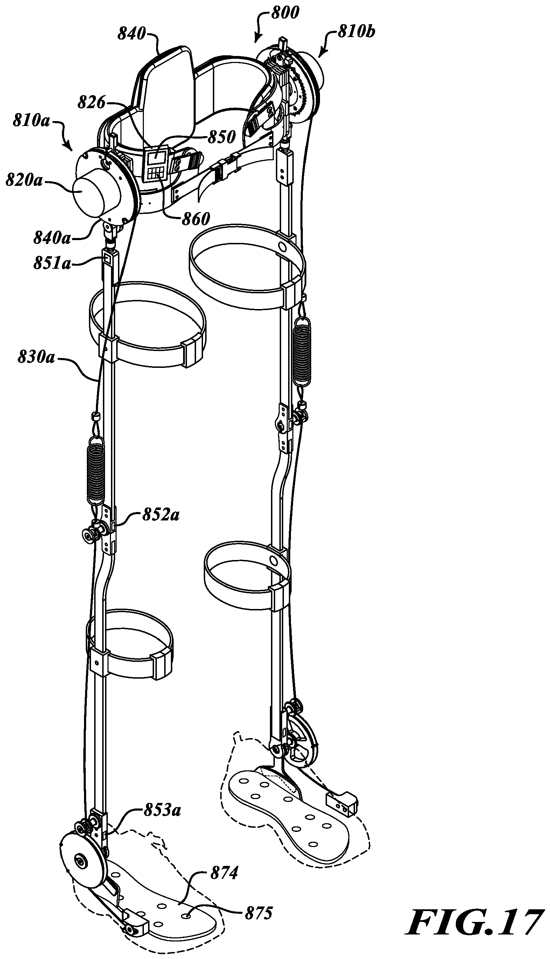

Figure 17 shows a motorized orthosis 800 that is similar to the ortheses of Figures 1-13 except as detailed below. The orthosis 800 includes a motorized energy storage apparatuses 810a, 810b (collectively "810"). The right energy storage apparatus 810a includes a motor 820a and a controller 826. The controller 826 is communicatively coupled to and can command the motor 820a to adjust the tension, if any, applied to an exotendon 830a. The controller 826 can also command the energy storage apparatus 810b. The energy storage apparatus 810a further includes a spool 840a about which the exotendon 830a can be wound. The motor 820a can be, without limitation, one or more stepper motors, DC motors, AC motors, combinations thereof, or other types of energizable devices capable of adjusting tension of exotendons.

The controller 826 is coupled to a belt assembly 840 and can generally include, without limitation, one or more central processing units, processing devices, microprocessors, digital signal processors (DSP), application-specific integrated circuits (ASIC), and the like. To store information, controllers also include one or more storage elements, such as volatile memory, non-volatile memory, read-only memory (ROM), random access memory (RAM), and the like. The controller 826 can include a display 850. The display 850 can include, without limitation, a LCD screen, a monitor, an analog display, a digital display (e.g., a light emitting diode display), or other devices suitable for displaying information. The display 850 can display the settings of the energy storage devices, force profiles, moment profiles, data collected by sensors (e.g., pressure applied by user, tension of exotendons, or the like), or any other information.

The controller 826 can store information. The term "information" includes, without limitation, one or more programs, executable code or instructions, operating instructions, combinations thereof, and the like. The controller 826 can store a wide range of different programs, including programs for adjusting the settings or the exotendon before and/or during use. The setting can be maintained until another program is selected by the user. In other embodiments, a program can be selected to provide variable performance based on, for example, signals from sensors (e.g., force sensors). In one exemplary non-limiting closed-loop embodiment, a program can be used to adjust loading of the exotendon 830a based on output from sensors. In open-loop embodiments, loading of the exotendon 830a is set by the user. For example, the user can select the tension in the exotendon 830a when the user stands vertically. The controller 826 can include an input device 860 (e.g., an input display, keyboard, touchpad, controller module, or any peripheral device for user input) and an internal power supply (e.g., one or more batteries or other type of power storage device) for powering the motor 820a, as well as other components of the orthosis 800.

Sensors 851 a, 852a, 853a can be in communication with the controller 826. The sensors 851 a, 852a, 853a can detect applied movement to analyze characteristics of the gait (e.g., weight transfer, timing of heel strikes, cadence, or the like), gait pattern, or the like and can be accelerometers, gyroscopes, or other types of motion sensors. Additionally or alternatively, one or more of the sensors 851 a, 852a, 853a can be force sensors (e.g., torque sensors, moment sensors, etc.) capable of generating an electrical output (e.g., signals) based on mechanical input. In some embodiments, a foot platform 874 includes sensors 875 in the form of pressure sensors. The feedback from the sensors 875 are used to adjust the settings of the orthosis 800.

Unless the context requires otherwise, throughout the specification and claims which follow, the word "comprise" and variations thereof, such as, "comprises" and "comprising" are to be construed in an open, inclusive sense, that is as "including, but not limited to."

As used in this specification and the claims, the singular forms "a," "an," and "the" include plural referents unless the content clearly dictates otherwise. It should also be noted that the term "or" is generally employed in its sense including "and/or" unless the context clearly dictates otherwise. The orthoses disclosed herein can be used or modified to be used with different body parts, include the legs, arms, fingers, or the like. Additionally, the orthosis can be combined with prosthesis or features described herein can be applied to a prosthesis. The components, configurations, and/or characteristics can be selected to achieve the desired amount of assistance.

The various embodiments described above can be combined to provide further embodiments. Aspects of the embodiments can be modified, if necessary to employ concepts of the various patents, applications, and publications to provide yet further embodiments. The orthoses can be modified to provide desired functionality, comfort, or the like. U.S. Patent No. 7,549,969 discloses various types of orthoses, apparatuses, frameworks, springs, joints, cables, and the like that can be incorporated into the embodiments disclosed herein. Besides, a wide range of different types of sensors, controllers, tensioning mechanisms, pylons, liner systems, puller units, that can be incorporated into the embodiments disclosed herein. In certain embodiments, the embodiments shown in Figures 1-13 and 17 include a tensioning mechanism or puller unit that can automatically adjust the tension of the exotendon 5.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

## Claims

1. A system (100; 800), comprising:
a belt assembly (8; 13-17; 104; 840) configured to be secured to a user's body; an energy storage apparatus (110; 810) coupled to the belt assembly (8; 13-17; 104; 840) and including an exotendon (5; 130a; 130b; 830); and
an articulatable leg frame (1-4; 112) including an upper end (1; 113a; 113b) rotatably coupled to the belt assembly (8; 13-17; 104; 840) and a lower end (4; 115a; 115b) coupleable to a lower portion of a leg of a user, the exotendon (5; 130a; 130b; 830a) is coupled to the adjustment device (9; 149a; 289a; 290a) and to the lower end (4; 115a; 115b) such that the exotendon (5; 130a; 130b; 830a) stores energy as the leg moves posteriorly and the exotendon (5; 130a; 130b; 830a) releases stored energy to help move the leg move anteriorly;
**characterised in that** said energy storage apparatus (110; 810) further comprises an adjustment device (9; 149a; 289a; 290a) configured to selectively adjust pretension in said exotendon (5: 130a; 130b; 830a).

2. The system (100; 800) of claim 1, wherein the energy storage apparatus (110; 810) has an unlocked state and a locked state, the energy storage apparatus (110; 810) in the unlocked state allows contraction of the exotendon (5; 130a; 130b; 830a), and the energy storage apparatus (110; 810) in the locked state holds the exotendon (5; 130a; 130b; 830a) in an extended state.

3. The system (100; 800) of claim 1, wherein the energy stored by the exotendon (5; 130a; 130b; 830a) increases as the leg moves posteriorly relative to the user's torso and the energy storage apparatus (110; 810) releases the stored energy to help move the leg anteriorly at the beginning of leg swing.

4. The system (100; 800) of claim 1, wherein the adjustment device (9; 149a; 289a; 290a) is manually operatable to adjust tension in the exotendon (5; 130a; 130b; 830a) while the system is worn by the user.

5. The system (100; 800) of claim 1, wherein the adjustment device (9; 149a; 289a; 290a) includes a one-way ratchet mechanism.

6. The system (100; 800) of claim 1, wherein the adjustment device (9; 149a; 289a; 290a) is capable of changing the tension in the exotendon (5; 130a; 130b; 830a) by at least about 10 percent.

7. The system (100; 800) of claim 1, wherein the articulatable leg frame (1-4; 112) is configured to provide a ratio of a minimum tensile force on the exotendon (5; 130a; 130b; 830a) to a maximum tensile force on the exotendon (5; 130a; 130b; 830a), wherein the ratio is in a range of about 3 to about 20 during normal walking.

8. The system (100; 800) of claim 1, wherein a maximum tensile force on the exotendon (5; 130a; 130b; 830a) is in a range about 2.1 kg-force meter (15 lb_{f}) to about 5.5 kg-force meter (40 lb_{f}) during normal walking.

9. The system (100; 800) of claim 1, wherein a minimum tensile force on the exotendon (5; 130a; 130b; 830a) is less than 2.1 kg-force meter (15 lb_{f}) during normal walking.

10. The system (100; 800) of claim 1, wherein the exotendon (5; 130a; 130b; 830a) extends along most of a length of the user's leg and stores most of the energy in a tension spring (6; 22; 23; 227a) of the exotendon (5; 130a; 130b; 830a).

11. The system (100; 800) of claim 1, further comprising a ball and socket joint (294a) that rotatably couples the upper end (1; 113a; 113b) of the articulatable leg frame (1-4; 112) to the energy storage apparatus (110; 810).

12. The system (100; 800) of claim 1, wherein the articulatable leg frame (1-4; 112) includes an ankle pulley (4) and a foot platform (7; 174a), wherein a portion of the exotendon (5; 130a; 130b; 830a) extends anteriorly from the ankle pulley (4) to the foot platform (7; 174a) and is positioned to be substantially parallel to a bottom of the user's foot when the user's foot is on the foot platform (7; 174a) and the ankle pulley (4) is adjacent to the user's ankle.

13. The system (100; 800) of claim 1, wherein the system is an orthosis or a prosthesis.

14. The system (100; 800) of claim 1, wherein the belt assembly (8; 13-17; 104; 840) includes
a first arm (14; 122a; 502a) having a first mounting end (507a),
a second arm (15; 122b; 502b) having a second mounting end (507b),
a back portion (13; 124) coupled to the first mounting end (507a) of the first
arm (14; 122a; 502a) and the second mounting end (507b) of the second arm (15; 122b; 502b), the back portion (124) having a positioner (503) extending vertically along the user's back when the first arm (14; 122a; 502a) and the second arm (15; 122b; 502b) wrap around the user's body.

15. The apparatus (100; 800) of claim 14, wherein the belt assembly (8; 13-17; 104; 840) includes a belt main body (500) and padding (499), the belt main body (500) comprises a material that is less compliant than material of the padding (499), wherein the padding (499) is detachably coupled to the belt main body (500).

## Patentansprüche

1. System (100; 800), umfassend:
eine Gurtanordnung (8; 13-17; 104; 840), die dafür konfiguriert ist, am Körper eines Benutzers gesichert zu werden;
eine Energiespeichervorrichtung (110; 810), die mit der Gurtanordnung (8; 13-17; 104; 840) verbunden ist und eine Exosehne (5; 130a; 130b;) beinhaltet, und
einen abknickbaren Beinrahmen (1-4; 112) beinhaltend ein oberes Ende (1; 113a; 113b), das drehbar mit der Gurtanordnung (8; 13-17; 104; 840) verbunden ist, und ein unteres Ende (4; 115a; 115b), das mit einem unteren Abschnitt eines Beins eines Benutzers verbindbar ist, wobei die Exosehne (5; 130a; 130b; 830a) mit der Einstellvorrichtung (9; 149a; 289a; 290a) und mit dem unteren Ende (4; 115a; 115b) so verbunden ist, dass die Exosehne (5; 130a; 130b; 830a) Energie speichert, während sich das Bein nach posterior bewegt, und die Exosehne (5; 130a; 130b; 830a) gespeicherte Energie freisetzt, um die Bewegung des Beins nach anterior zu unterstützen;
**dadurch gekennzeichnet, dass** besagte Energiespeichervorrichtung (110; 810) ferner eine Einstellvorrichtung (9; 149a; 289a; 290a) umfasst, die dafür konfiguriert ist, die Vorspannung in besagter Exosehne (5; 130a; 130b; 830a) selektiv einzustellen.

2. System (100; 800) nach Anspruch 1, worin die Energiespeichervorrichtung (110; 810) einen entriegelten Zustand und einen verriegelten Zustand aufweist, die Energiespeichervorrichtung (110; 810) im entriegelten Zustand ein Zusammenziehen der Exosehne (5; 130a; 130b; 830a) erlaubt und die Energiespeichervorrichtung (110; 810) im verriegelten Zustand die Exosehne (5; 130a; 130b; 830a) in einem gestreckten Zustand hält.

3. System (100; 800) nach Anspruch 1, worin die von der Exosehne (5; 130a; 130b; 830a) gespeicherte Energie zunimmt, während sich das Bein nach posterior relativ zum Oberkörper des Benutzers bewegt, und die Energiespeichervorrichtung (110; 810) die gespeicherte Energie freisetzt, um die Bewegung des Beins nach anterior zu Beginn des Beinschwungs zu unterstützen.

4. System (100; 800) nach Anspruch 1, worin die Einstellvorrichtung (9; 149a; 289a; 290a) manuell betätigbar ist, um die Spannung in der Exosehne (5; 130a; 130b; 830a) einzustellen, während das System vom Benutzer getragen wird.

5. System (100; 800) nach Anspruch 1, worin die Einstellvorrichtung (9; 149a; 289a; 290a) einen Einweg-Ratschenmechanismus beinhaltet.

6. System (100; 800) nach Anspruch 1, worin die Einstellvorrichtung (9; 149a; 289a; 290a) fähig ist, die Spannung in der Exosehne (5; 130a; 130b; 830a) um mindestens etwa 10 Prozent zu ändern.

7. System (100; 800) nach Anspruch 1, worin der abknickbare Beinrahmen (1-4; 112) dafür konfiguriert ist, ein Verhältnis einer Mindestzugkraft an der Exosehne (5; 130a; 130b; 830a) zu einer Höchstzugkraft an der Exosehne (5; 130a; 130b; 830a) bereitzustellen, worin das Verhältnis in einem Bereich von etwa 3 bis etwa 20 während des normalen Gehens liegt.

8. System (100; 800) nach Anspruch 1, worin eine Höchstzugkraft an der Exosehne (5; 130a; 130b; 830a) in einem Bereich von etwa 2,1 kgfm (15 lbf) bis etwa 5,5 kgfm (40 lbf) während des normalen Gehens liegt.

9. System (100; 800) nach Anspruch 1, worin eine Mindestzugkraft an der Exosehne (5; 130a; 130b; 830a) weniger als 2,1 kgfm (15 lbf) während des normalen Gehens beträgt.

10. System (100; 800) nach Anspruch 1, worin sich die Exosehne (5; 130a; 130b; 830a) den größten Teil einer Länge des Beins des Benutzers entlang erstreckt und den größten Teil der Energie in einer Spannungsfeder (6; 22; 23; 227a) der Exosehne (5; 130a; 130b; 830a) speichert.

11. System (100; 800) nach Anspruch 1, ferner umfassend ein Kugelgelenk (294a), das das obere Ende (1; 113a; 113b) des abknickbaren Beinrahmens (1-4; 112) drehbar mit der Energiespeichervorrichtung (110; 810) verbindet.

12. System (100; 800) nach Anspruch 1, worin der abknickbare Beinrahmen (1-4; 112) eine Sprunggelenk-Umlenkrolle (4) und eine Fußplattform (7; 174a) beinhaltet, worin sich ein Abschnitt der Exosehne (5; 130a; 130b; 830a) nach anterior von der Sprunggelenk-Umlenkrolle (4) zur Fußplattform (7; 174a) erstreckt und positioniert ist, um im Wesentlichen parallel zu einer Unterseite des Fußes des Benutzers zu sein, wenn sich der Fuß des Benutzers auf der Fußplattform (7; 174a) befindet und die Sprunggelenk-Umlenkrolle (4) neben dem Sprunggelenk des Benutzers ist.

13. System (100; 800) nach Anspruch 1, worin das System eine Orthese oder eine Prothese ist.

14. System (100; 800) nach Anspruch 1, worin die Gurtanordnung (8; 13-17; 104; 840) Folgendes beinhaltet:
einen ersten Arm (14; 122a; 502a) mit einem ersten Befestigungsende (507a),
einen zweiten Arm (15; 122b; 502b) mit einem zweiten Befestigungsende (507b),
ein Rückenteil (13; 124), das mit dem ersten Befestigungsende (507a) des ersten Arms (14; 122a; 502a) und dem zweiten Befestigungsende (507b) des zweiten Arms (15; 122b; 502b) verbunden ist, wobei das Rückenteil (124) eine Positioniereinrichtung (503) aufweist, die sich vertikal entlang des Rückens des Benutzers erstreckt, wenn sich der erste Arm (14; 122a; 502a) und der zweite Arm (15; 122b; 502b) um den Körper des Benutzers herum wickeln.

15. Vorrichtung (100; 800) nach Anspruch 14, worin die Gurtanordnung (8; 13-17; 104; 840) einen Gurthauptkörper (500) und Polsterung (499) beinhaltet, worin der Gurthauptkörper (500) ein Material umfasst, das weniger nachgiebig als Material der Polsterung (499) ist, worin die Polsterung (499) abnehmbar mit dem Gurthauptkörper (500) verbunden ist.

## Revendications

1. Un système (100; 800), comprenant:
un ensemble courroie (8; 13-17; 104; 840) configuré pour être fixé au corps d'un utilisateur;
un appareil de stockage d'énergie (110; 810) raccordé à l'ensemble courroie (8; 13-17; 104; 840) et incluant un exotendon (5; 130a; 130b;), et
une structure articulée pour jambe (1-4; 112) incluant une extrémité supérieure (1; 113a; 113b) raccordée de manière à pouvoir tourner à l'ensemble courroie (8; 13-17; 104; 840) et une extrémité inférieure (4; 115a; 115b) pouvant être raccordée à une partie inférieure d'une jambe d'un utilisateur, l'exotendon (5; 130a; 130b; 830a) est raccordé au dispositif d'ajustement (9; 149a; 289a; 290a) et à l'extrémité inférieure (4; 115a; 115b) de telle sorte que l'exotendon (5; 130a; 130b; 830a) emmagasine l'énergie lorsque la jambe se déplace vers l'arrière et l'exotendon (5; 130a; 130b; 830a) libère l'énergie emmagasinée pour aider la jambe à se déplacer vers l'avant;
**caractérisé en ce que** ledit appareil de stockage d'énergie (110; 810) comprend en outre un dispositif d'ajustement (9; 149a; 289a; 290a) configuré pour ajuster de manière sélective la pré-tension dans ledit exotendon (5: 130a; 130b; 830a).

2. Le système (100; 800) selon la revendication 1, dans lequel l'appareil de stockage d'énergie (110; 810) a un état déverrouillé et un état verrouillé, l'appareil de stockage d'énergie (110; 810) à l'état déverrouillé permet la contraction de l'exotendon (5; 130a; 130b; 830a) et l'appareil de stockage d'énergie (110; 810) à l'état verrouillé maintient l'exotendon (5; 130a; 130b; 830a) dans un état étendu.

3. Le système (100; 800) selon la revendication 1, dans lequel l'énergie emmagasinée par l'exotendon (5; 130a; 130b; 830a) augmente lorsque la jambe se déplace vers l'arrière par rapport au torse de l'utilisateur et que l'appareil de stockage d'énergie (110; 810) libère l'énergie emmagasinée pour aider la jambe à se déplacer vers l'avant au début du balancement de la jambe.

4. Le système (100; 800) selon la revendication 1, dans lequel le dispositif d'ajustement (9; 149a; 289a; 290a) peut être actionner manuellement pour ajuster la tension dans l'exotendon (5; 130a; 130b; 830a) pendant que le système est porté par l'utilisateur.

5. Le système (100; 800) selon la revendication 1, dans lequel le dispositif d'ajustement (9; 149a; 289a; 290a) inclut un mécanisme à cliquet unidirectionnel.

6. Le système (100; 800) selon la revendication 1, dans lequel le dispositif d'ajustement (9; 149a; 289a; 290a) est capable de modifier la tension dans l'exotendon (5; 130a; 130b; 830a) d'au moins environ 10 pour cent.

7. Le système (100; 800) selon la revendication 1, dans lequel la structure articulée pour jambe (1-4; 112) est configurée pour fournir un rapport d'une force de traction minimale sur l'exotendon (5; 130a; 130b; 830a) à une force de traction maximale sur l'exotendon (5; 130a; 130b; 830a), dans lequel le rapport est dans une plage comprise entre environ 3 et environ 20 lors de la marche normale.

8. Le système (100; 800) selon la revendication 1, dans lequel une force de traction maximale sur l'exotendon (5; 130a; 130b; 830a) est dans une plage comprise entre environ 2,1 kg force mètre (15 lbf) et environ 5,5 kg force mètre (40 lbf) lors de la marche normale.

9. Le système (100; 800) selon la revendication 1, dans lequel une force de traction minimale sur l'exotendon (5; 130a; 130b; 830a) est inférieure à 2,1 kg force mètre (15 lbf) au cours d'une marche normale.

10. Le système (100; 800) selon la revendication 1, dans lequel l'exotendon (5; 130a; 130b; 830a) s'étend le long de la majorité de la longueur de la jambe de l'utilisateur et emmagasine la majorité de l'énergie dans un ressort de tension (6; 22; 23; 227a) de l'exotendon (5; 130a; 130b; 830a).

11. Le système (100; 800) selon la revendication 1, comprenant en outre un joint à rotule (294a) qui raccorde de manière à pouvoir tourner l'extrémité supérieure (1; 113a; 113b) de la structure articulée pour jambe (1-4; 112) à l'appareil de stockage d'énergie (110; 810).

12. Le système (100; 800) selon la revendication 1, dans lequel la structure articulée pour jambe (1-4; 112) inclut une poulie pour cheville (4) et une plateforme pour pied (7; 174a), dans lequel une partie de l'exotendon (5; 130a; 130b; 830a) s'étend vers l'avant depuis la poulie pour cheville (4) vers la plateforme pour pied (7; 174a) et est positionnée pour être sensiblement parallèle à une partie inférieure du pied de l'utilisateur lorsque le pied de l'utilisateur est sur la plateforme pour pied (7; 174a) et la poulie pour cheville (4) est adjacente à la cheville de l'utilisateur.

13. Le système (100; 800) selon la revendication 1, dans lequel le système est une orthèse ou une prothèse.

14. Le système (100; 800) selon la revendication 1, dans lequel l'ensemble courroie (8; 13-17; 104; 840) inclut:
un premier bras (14; 122a; 502a) ayant une première extrémité de montage (507a),
un deuxième bras (15; 122b; 502b) ayant une deuxième extrémité de montage (507b),
une partie dos (13; 124) raccordée à la première extrémité de montage (507a) du premier bras (14; 122a; 502a) et la deuxième extrémité de montage (507b) du deuxième bras (15; 122b; 502b), la partie dos (124) ayant un positionneur (503) s'étendant verticalement le long du dos de l'utilisateur lorsque le premier bras (14; 122a; 502a) et le deuxième bras (15; 122b; 502b) s'enroulent autour du corps de l'utilisateur.

15. L'appareil (100; 800) selon la revendication 14, dans lequel l'ensemble courroie (8; 13-17; 104; 840) inclut un corps principal de la courroie (500) et un rembourrage (499), le corps principal de la courroie (500) comprend un matériau qui est moins souple que le matériau du rembourrage (499), dans lequel le rembourrage (499) est raccordé de manière amovible au corps principal de la courroie (500).
